Europäisches Patentamt

European Patent Office

Office européen des brevets

(11) Veröffentlichungsnummer: **0 222 283 B1**

(12) **EUROPÄISCHE PATENTSCHRIFT**

(45) Veröffentlichungstag der Patentschrift: **30.12.92**

(21) Anmeldenummer: **86115175.1**

(22) Anmeldetag: **01.11.86**

Die Akte enthält technische Angaben, die nach dem Eingang der Anmeldung eingereicht wurden und die nicht in dieser Patentschrift enthalten sind.

(51) Int. Cl.5: **C07K 5/00**, C07K 5/02, A61K 37/02, C07D 403/12, C07D 235/14

(54) **Aminosäurederivate.**

(30) Priorität: **15.11.85 DE 3540495**

(43) Veröffentlichungstag der Anmeldung: **20.05.87 Patentblatt 87/21**

(45) Bekanntmachung des Hinweises auf die Patenterteilung: **30.12.92 Patentblatt 92/53**

(84) Benannte Vertragsstaaten: **AT BE CH DE ES FR GB IT LI NL SE**

(56) Entgegenhaltungen:
**EP-A- 81 783**
**EP-A- 118 223**
**EP-A- 0 156 322**

**AGRICULTURAL AND BIOLOGICAL CHEMISTRY, Band 41, Nr. 5, 1977, Seiten 811-818; K. MAEKAWA et al.: "Synthesis and biological activities of benzimidazole derivatives from peptides"**

(73) Patentinhaber: **MERCK PATENT GESELL-SCHAFT MIT BESCHRÄNKTER HAFTUNG Frankfurter Strasse 250 Postfach 4119 W-6100 Darmstadt(DE)**

(72) Erfinder: **Raddatz, Peter, Dr.**
**Grafenstrasse 37**
**W-6100 Darmstadt(DE)**
Erfinder: **Hölzemann, Günter, Dr.**
**Weedring 5**
**W-6104 Seeheim 1(DE)**
Erfinder: **Schmitges, Claus J., Dr.**
**Karolinger Strasse 5**
**W-6114 Gross-Umstadt(DE)**
Erfinder: **Minck, Klaus Otto, Dr.**
**Büchestrasse 8**
**W-6108 Ober-Ramstadt(DE)**

EP 0 222 283 B1

## Beschreibung

Die Erfindung betrifft neue Aminosäurederivate der Formel I

X-Phe-His-AHCP-B-NH-CHR-Q          I

worin

| | | |
|---|---|---|
| X | Alkoxycarbonyl mit 1-4 C-Atomen, | |
| B | fehlt oder Ile oder Leu, | |
| R | H oder A, | |
| Q | Benzimidazol-2-yl, Imidazo[4,5-b]pyridin-2-yl oder Imidazo[4,5-c]pyridin-2-yl und | |
| A | Alkyl mit 1-4 C-Atomen bedeuten, | |

sowie deren Salze.

Ähnliche Verbindungen sind aus der EP-A-77028 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurd gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich nöhere Konzentrationen dieser Verbindung notwendig.

Die Verbindungen können als Arzneimittelwirkstoffe in der Human-und Veterinärmedizin eingesetzt werden, insbesondere zur Prophylaxe und zur Behandlung von Herz-, Kreislauf-und Gefäßkrankheiten, vor allem von Hypertonie, Herzinsuffizienz und Hyperaldosteronismus, Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor-und nachstehend aufgeführten Abkürzungen von Aminosäureresten stehen für die Reste -NH-CHR-CO-(worin R die für jede Aminosäure bekannte spezifische Bedeutung hat) folgender Aminosäuren:

His Histidin
Ile Isoleucin
Leu Leucin
Phe Phenylalanin
Ferner bedeutet nachstehend:
BOC tert.-Butoxycarbonyl
imi-BOM Benzyloxymethyl in 1-Stellung des Imidazolrings
CBZ Benzyloxycarbonyl

DNP 2,4-Dinitrophenyl
imi-DNP 2,4-Dinitrophenyl in 1-Stellung des Imidazolrings
FMOC 9-Fluorenylmethoxycarbonyl.

Sofern die vorstehend gennanten Aminosäuren in mehreren enantiomeren Formen auftreten können, so sind vor-und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Vor-und nachstehend haben die Reste bzw. Parameter X, B, die bei den Formeln I, II oder III angegebenen Bedeutungen, falls nicht ausdrücklich etwas anderes angegeben ist.

In den vorstehenden Formeln hat A 1,2,3 oder 4 C-Atome. A bedeutet vorzugsweise Methyl, weiterhin Ethyl, Propyl, Isopropyl, Butyl, Isobutyl, sek.-Butyl oder tert.-Butyl.

X bedeutet vorzugsweise BOC.

Der Rest Q ist vorzugsweise Benzimidazol-2-yl, oder Imidazo[4,5-c]pyridin-2-yl.

"AHCP", abgeleitet von 4-Amino-3-hydroxy-5-cyclohexylpentansäure, bedeutet, -NH-CH-(Cyclohexylmethyl)-CHOH-$CH_2$-CO-

Die Gruppe AHCP besitzt zwei chirale Zentren. Die Verbindungen der Formel I können daher in verschiedenen -optisch-inaktiven oder optisch-aktiven -Formen vorkommen. Die Formel I umschließt alle diese Formen. Die 3S-Hydroxy-4S-amino-Enantiomeren sind bevorzugt Die Abkürzung AHCP bezieht sich immer auf diese 3S, 4S-Form.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt.

Die Verbindungen der Formel I und auch die Ausgangsstoffe zu ihrer Herstellung werden im übrigen nach an sich bekannten Methoden hergestellt, wie sie in der Literatur (z. B. in den Standardweken wie Houben-Weyl, Methoden der organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Vorzugsweise werden die Verbindungen der Formel I erhalten, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrere freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N-(im)-$R^8$-His-Gruppe (worin $R^8$ eine Aminoschutzgruppe bedeutet, z. B. BOM oder DNP) enthalten.

Ferner sind Ausgangsstoffe bevorzugt, die an Stelle des H-Atoms einer Hydroxygrupe eine Hydroxyschutzgruppe tragen, z. B. solche der Formel X-Phe-His-NH-CH(cyclohexylmethyl)-CHOR*-$CH_2$-CO-B-NH-CHR-Q, worin R* eine Hydroxyschutzgruppe bedeutet.

Es können auch mehrere -gleiche oder verschiedene -geschützte Amino -und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind nachdem die gewünschte chemische Reaktion an anderen Stellen des Moleküls durchgeführt worden ist. Typisch für solche Gruppen sind insbesondere unsubstituierte oder substituierte Acyl-, Aryl-(z. B. 2,4-Dinitrophenyl), Aralkoxymethyl-(z. B. Benzyloxymethyl) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl-und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Aralkyloxycarbonyl wie CBZ ("Carbobenzoxy"), 4-Methoxybenzyloxycarbonyl, FMOC. Bevorzugte Aminoschutzgruppen sind DNP, BOM, CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl-oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe -z B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° - (Raumtemperatur).

Die FMOC-Gruppe kann z. B. bevorzugt mit einer etwa 5-bis 20%igen Lösung von Dimethylamin, Diethylamin oder Piperidin in DMF bei 15-30° abgespalten werden. Ein Abspaltung der DNP-Gruppe gelingt z. B. auch mit einer etwa 3-bis 10%igen Lösung von 2-Mercaptoethanol in DMF/Wasser bei 15-30°.

Hydrogenolytisch entfernbare Schutzgruppen - (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5- bis 10%igem Pd-C in Methanol bei 20-30°.

Die neuen Verbindungen der Formel I und ihre physiologisch unbedenklichen Salze können zur Herstellung pharmazeutischer Präparate verwendet werden, indem man sie zusammen mit mindestens einem Träger-oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human-oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen.

Zur rektalen Anwendung dienen Suppositorien, zur parenteralen Applikation Lösungen, vorzugsweise ölige oder wässerige Lösungen, ferner Suspensionen, Emulsionen oder Implantate. Fur die Applikation als Inhalations-Spray können Sprays verwendet werden, die den Wirkstoff entweder gelöst oder suspendiert in einem Treibgasgemisch (z. B. Fluorchlor-kohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb-und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoff kombination und Schwere der jeweiligen Erkrankung, welcher die Therapie gilt. Die parenterale Applikation ist bevorzugt.

Vor-und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Ein Gemisch von 400 mg 2-[1S-(3S-Hydroxy-4S-(N-tert.-butoxycarbonyl-L-phenylalanyl-N(im)-(2,4-dinitrophenyl)-L-histidyl-amino)-5-cyclohexylpentanoylamino)-3-methylbutyl]-benzimidazol ["2-(1S-BOC-Phe-imi-DNP-His-AHCP-amino-3-methylbutyl)-benzimidazol"; F. 130° (Zers.); erhältlich durch Reaktion von BOC-Ile-Oh mit o-Phenylendiamin zu N-BOC-Ile-o-phenylendiamin, Cyclisierung mit Essigsäure (5 Std. bei 65°) zu 2-(1S-BOC-amino-3-methylbutyl)-benzimidazol - (F. 211-213°), Abspaltung der BOC-Gruppe mit 4n HCl in Dioxan zu 2-(1S-Amino-3-methylbutyl)-benzimidazol (F. 240-242° (Zers.)), Reaktion mit BOC-AHCP-OH/DCCI/HOBt zu 2-(1S-BOC-AHCP-amino-3-methylbutyl)-benzimidazol (F. 180-182°), Abspaltung der BOC-Gruppe und Kondensation mit BOC-imi-DNP-His-OH zu 2-(1S-BOC-imi-DNP-His-AHCP-amino-3-methylbutyl)-benzimidazol (F. 140°
Zers.), erneute Abspaltung der BOC-Gruppe und
Reaktion mit BOC-Phe-OH], 860 mg 2-Mercaptoethanol, 10 ml DMF und 10 ml Wasser wird unter
Rühren bei 20° mit wässeriger $Na_2CO_3$-Lösung
auf pH 8 eingestellt und 2 Std. bei 20° gerührt.
Nach üblicher Aufarbeitung erhält man 2-[1S-(3S-
Hydroxy-4S-(N-tert.-butoxycarbonyl-L-phenylalanyl-
L-histidylamino)-5-cyclohexylpentanoylamino)-3-
methylbutyl]-benzimidazol ["2-(1S-BOC-Phe-His-
AHCP-amino-3-methylbutyl)-benzimidazol"], F.
140° (Zers.).

Analog erhält man durch Spaltung der entsprechenden imi-DNP-Derivate:
2-(BOC-Phe-His-AHCP-Leu-aminomethyl)-1H-
imidazo[4,5-b]-pyridin, Sinterpunkt 165 - 170°
2-(BOC-Phe-His-AHCP-Leu-aminomethyl)-1H-
imidazo[4,5-c]-pyridin, Sinterpunkt 110 -115°
2-(BOC-Phe-His-AHCP-aminomethyl)-benzimidazol,
Formiat, F 201° (Zers.)
2-(BOC-Phe-His-AHCP-aminomethyl)-1H-imidazo-
[4,5-b]pyridin, Sinterpunkt 185 -195°
2-(BOC-Phe-His-AHCP-aminomethyl)-1H-imidazo-
[4,5-c]pyridin Sinterpunkt 165 -170°
2-(1S-BOC-Phe-His-AHCP-amino-3-methylbutyl)-
1H-imidazo[4,5-b]pyridin, Sinterpunkt 130 -135°
2-(BOC-Phe-His-AHCP-Ile-aminomethyl)-
benzimidazol, F. 213° (Zers.)

Beispiel 2

Man löst 1 g 2-(1S-BOC-Phe-imi-BOM-His-
AHCP-amino-3-methylbutyl)-benzimidazol
- [erhältlich aus 2-(1S-AHCP-amino-3-methylbutyl)-
benzimidazol durch Kondensation mit BOC-Phe-
imi-BOM-His-OH] in 10 ml Methanol, hydriert an
0,5 g 10%ig. Pd-C bei 20° und 1 bar 3 Std. lang,
filtriert, dampft ein und erhält 2-(1S-BOC-Phe-His-
AHCP-amino-3-methylbutyl)-benzimidazol, F. 140°
(Zers.).

Analog erhält man aus den entsprechenden
imi-BOM-Derivaten die in Beispiel 1 angegebenen
Verbindungen.

Die nachstehenden Beispiele betreffen pharmazeutische Zubereitungen.

Beispiel A: Injektionsgläser

Eine Lösung von 100 g 2-(BOC-Phe-His-AHCP-
aminomethyl)-benzimidazol-formiat und 5 g Dinatriumhydrogenphosphat in 3 l zweifach destilliertem
Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg
Wirkstoff.

Beispiel B: Suppositorien

Man schmilzt ein Gemisch von 500 g 2-(1S-
BOC-Phe-His-AHCP-Ile-amino-3-methylbutyl)-
benzimidazol mit 100 g Sojalecithin und 1400 g
Kakaobutter, gießt in Formen und läßt erkalten.
Jedes Suppositorium enthält 500 mg Wirkstoff.

**Patentansprüche**

1. Aminosäurederivate der Formel I

   X-Phe-His-AHCP-B-NH-CHR-Q     I

   worin
   
   | | |
   |---|---|
   | X | Alkoxycarbonyl mit 1-4 C-Atomen, |
   | B | fehlt oder Ile oder Leu, |
   | R | H oder A, |
   | Q | Benzimidazol-2-yl, Imidazo[4,5-b]-pyridin-2-yl oder Imidazo[4,5-c]-pyridin-2-yl und |
   | A | Alkyl mit 1-4 C-Atomen |
   
   bedeuten
   sowie deren Salze.

2. 
   a) 2-(BOC-Phe-His-AHCP-Leu-aminomethyl)-1H-imidazo[4,5-b]pyridin.
   b) 2-(BOC-Phe-His-AHCP-Ile-aminomethyl)-
   benzimidazol.
   c) 2-(BOC-Phe-His-AHCP-Leu-aminomethyl)-1H-imidazo[4,5-c]pyridin.
   d) 2-(1S-BOC-Phe-His-AHCP-Ile-amino-3-
   methyl-butyl)-benzimidazol.
   e) 2-(1S-BOC-Phe-His-AHCP-amino-3-
   methyl-butyl)-1H-imidazo[4,5-b]pyridin.
   f) 2-(1S-BOC-Phe-His-AHCP-amino-3-
   methyl-butyl)-benzimidazol.

3. Verfahren zur Herstellung eines Aminosäurederivats der Formel I sowie von seinen Salzen,
   dadurch gekennzeichnet, daß man es aus einem seiner funktionellen Derivate durch Behandeln mit einem solvolysierenden oder hydrogenolysierenden Mittel in Freiheit setzt.

4. Verfahren zur Herstellung pharmazeutischer
   Zubereitungen, dadurch gekennzeichnet, daß
   man eine Verbindung der Formel I und/oder
   eines ihres physiologisch unbedenklichen Salze zusammen mit mindestens einem festen,
   flüssigen oder halbflüssigen Träger- oder Hilfsstoff und gegebenenfalls in Kombination mit
   einem oder mehreren weiteren Wirkstoff(en) in
   eine geeignete Dosierungsform bringt.

5. Pharmazeutische Zubereitung, gekennzeichnet
   durch einen Gehalt an mindestens einer Verbindung der Formel I und/oder einem ihrer
   physiologisch unbedenklichen Salze.

6. Verbindungen der Formel I und deren physiologisch unbedenklichen Salze zur Bekämpfung von Krankheiten.

7. Verwendung von Verbindungen der Formel I oder von deren physiologisch unbedenklichen Salzen zur Herstellung eines Arzneimittels zur Bekämpfung der reninabhängigen Hypertension oder des Hyperaldosteronismus.

**Claims**

1. Aminoacid derivatives of formula I

   X-Phe-His-AHCP-B-NH-CHR-Q     I

   wherein
   X       is alkoxycarbonyl with 1-4 C atoms,
   B       is absent or is Ile or Leu,
   R       is H or A,
   Q       is benzimidazol-2-yl, imidazo[4,5-b]-pyridin-2-yl or imidazo[4,5-c]pyridin-2-yl and
   A       is alkyl with 1-4 C atoms,
   an their salts.

2.
   a)    2-(BOC-Phe-His-AHCP-Leu-aminomethyl)-1H-imidazo[4,5-b]pyridine.
   b)    2-(BOC-Phe-His-AHCP-Ile-aminomethyl)-benzimidazole.
   c)    2-(BOC-Phe-His-AHCP-Leu-aminomethyl)-1H-imidazo[4,5-c]pyridine.
   d)    2-(1S-BOC-Phe-His-AHCP-Ile-amino-3-methyl-butyl)-benzimidazole.
   e)    2-(1S-BOC-Phe-His-AHCP-amino-3-methyl-butyl)-1H-imidazo[4,5-b]pyridine.
   f)    2-(1S-BOC-Phe-His-AHCP-amino-3-methyl-butyl)-benzimidazole.

3. Process for the preparation of an aminoacid derivative of the formula I and of its salts, characterized in that it is liberated from one of its functional derivatives by treatment with a solvolysing or hydrogenolysing agent.

4. Process for the preparation of pharmaceutical formulations, characericed in that a compound of the formula I and/or one of its physiologically acceptable salts is converted into a suitable dosage form together with at least one solid, liquid or semi-liquid vehicle or auxiliary and, where appropriate, in combination with one or more other compound(s).

5. Pharmaceutical formulation characterized by containing at least one compound of the formula I and/or one of its physiologically acceptable salts.

6. Compounds of the formula I and their physiologically acceptable salts for combating diseases.

7. Use of compounds of the formula I or of their physiologically acceptable salts for the preparation of a medicament for combating renin-dependent hypertension or hyperaldosteronism.

**Revendications**

1. Dérivés d'aminoacides de formule I :

   X-Phe-His-AHCP-B-NH-CHR-Q     I

   dans laquelle
   X       représente un groupe alcoxycarbonyle en C 1-C 4,
   B       manque ou représente Ile ou Leu,
   R       représente H ou A,
   Q       représente un groupe benzimidazole-2-yle, imidazo[4,5-b]pyridine-2-yle ou imidazo[4,5-c]pyridine-2-yle, et
   A       représente un groupe alkyle en C 1-C 4
   et leurs sels.

2.
   a) la 2-(BOC-Phe-His-AHCP-Leu-aminométhyl)-1H-imidazo[4,5-b]pyridine.
   b) le 2-(BOC-Phe-His-AHCP-Ile-aminométhyl)-benzimidazole.
   c) la 2-(BOC-Phe-His-AHCP-Leu-aminométhyl)-1H-imidazo[4,5-c]pyridine.
   d) le 2-(1S-BOC-Phe-His-AHCP-Ile-amino-3-méthyl-butyl)-benzimidazole.
   e) la 2-(1S-BOC-Phe-His-AHCP-amino-3-méthyl-butyl)-1H-imidazo[4,5-b]pyridine.
   f) le 2-(1S-BOC-Phe-His-AHCP-amino-3-méthyl-butyl)-benzimidazole.

3. Procédé de préparation d'un dérivé d'aminoacide de formule I et de ses sels, caractérisé en ce que, partant de l'un de ses dérivés fonctionnels, on le libère en traitant par un agent solvolysant ou hydrogénolysant.

4. Procédé de préparation de compositions pharmaceutiques, caractérisé en ce que l'on met un composé de formule I et/ou l'un de ses sels acceptables pour l'usage pharmaceutique sous une forme de dosage appropriée avec au moins un véhicule ou produit auxiliaire solide, liquide ou semi-liquide et le cas échéant en combinaison avec une ou plusieurs autres substances actives.

5. Composition pharmaceutique, caractérisée en ce qu'elle contient au moins un composé de formule I et/ou l'un de ses sels acceptables pour l'usage pharmaceutique.

6. Composés de formule I et leurs sels acceptables pour l'usage pharmaceutique, pour le traitement de maladies.

7. Utilisation des composés de formule I ou de leurs sels acceptables pour l'usage pharmaceutique pour la préparation d'un médicament pour le traitement de l'hypertension ou de l'hyperaldostéronisme en relation avec la rénine.